# EUROPEAN PATENT APPLICATION

(11) **EP 0 716 833 A2**
(43) Date of publication of application: **19.06.1996**
(21) Application number: 95308976.0
(22) Date of filing: 11.12.1995
(51) Int. Cl.: A61B 19/00

(54) **Method and apparatus for sealing a body site**

(30) Priority: 14.12.1994 US 356073
(71) Applicant: GLOBAL THERAPEUTICS INC., Broomfield, Colorado 80020 (US)
(72) Inventor: Horn, Joseph B., Niwot, Colorado 80503 (US); Petrarca, Daniel J., Boulder, Colorado 80303 (US); Dorros, Gerald, Fox Point, Wisconsin 53217 (US); Ferguson III, James J., Houston, Texas 77030 (US); Wiesner, Steven P., Cupertino, California 95014 (US)
(74) Representative: Enskat, Michael Antony Frank

(57) **Abstract**

An apparatus for sealing a puncture in a body includes an insertion tip (64) for entering the puncture. A stop (68) connected to said tip (64) is arranged to lie adjacent to the puncture when the apparatus is used for sealing the puncture.

A trunk (28) is connected to the stop (68) for manual manipulation by a user and a sealant (34) is delivered to the trunk (48) from a mixing chamber (96).

The sealant (34) is urged to exit an outlet (36) in the trunk (28) and the stop (68) prevents said sealant (34) from entering the puncture but the stop (68) is removed from adjacent the puncture after said sealant (34) is located adjacent the puncture.

## Description

The present invention relates to method and apparatus for sealing, including for use in sealing an arterial puncture produced, for example, by an arterial catheterization procedure.

Upon termination of a catheterization process where the catheter is removed from, for example, an artery, the artery puncture caused by the catheter must be properly closed to prevent bleeding complications. For example, such bleeding complications lead to the formation of a hematoma which, in turn, can necessitate surgery.

To date, currently available hemostatic devices for sealing such arterial punctures require lengthy procedures (e.g., 20-40 minutes) and/or are uncomfortable for the patient (e.g., cauterization of the puncture). Further, such devices may require accurately positioning a patch on the puncture or a plug into the puncture. Inaccurate positioning may lead to bleeding from the puncture, occlusion of blood flow in the artery and/or a portion of the patch or plug dislodging into the artery.

It is, therefore, desirable to have a hemostatic device which substantially alleviates the above shortcomings and is both cost effective and simple to use. In particular, it is desirable to provide a hemostatic method and apparatus which, when used, requires substantially less time with less patient discomfort and provides the necessary safety for the patient.

The present invention relates to a method and apparatus for sealing to control bleeding or other body fluid flow in conjunction with a medical procedure including the patching of a puncture in a body lumen, such as an artery having a puncture to be sealed after the removal of an arterial catheter. The present invention is directed to a hemostatic apparatus which enters a tissue opening providing access to such a puncture and seals the puncture by injecting a gelatinous bioabsorptive sealant, having the property of being tissue adhesive, into tissue adjacent to the lumen puncture. In doing this, the sealant is prevented from entering the lumen by providing a covering or barrier for the lumen puncture while the sealant is being positioned about the puncture. Preferably also, the sealant is delivered from the apparatus at a predetermined distance from the puncture.

As a further aspect of one embodiment of the present invention, the capability is provided to indicate when the apparatus is properly located in preparation for sealant injection. More precisely, a guide thread or wire is used with the apparatus. When an end of the wire has been threaded into the lumen puncture, then an opposite end can be inserted through a guide bore traversing a longitudinal axis of the apparatus. This provides a way to guide the apparatus to the lumen puncture. That is, an insertion tip of the apparatus is guided into the lumen puncture until an insertion stop provides resistance to further entry into the puncture, thereby indicating to a health care giver using the apparatus that it is properly positioned.

In one embodiment, the sealant used is a composition that includes thrombin and fibrinogen and is formed, immediately prior to injection, from a mixing of the components. Note that this sealant can form an effective arterial patch in less than five minutes and typically less than two minutes.

Once the apparatus is in place and the sealant is being injected such that it flows toward and surrounds the insertion stop, the practitioner or health care giver need only apply manual external pressure during the use of the apparatus and for a minimal amount of time thereafter (e.g., less than two minutes) while allowing the sealant to firmly adhere to the tissue adjacent to the puncture.

With regard to the application of manual external pressure, in one embodiment, this pressure is provided by the practitioner directly by using his/her fingers against the skin adjacent to the puncture. In another embodiment, a pressure applicator device engages the patient's skin and a force is applied to the skin by the device. The pressure applicator device is also able to forcefully engage the patient's skin after removal of the apparatus that delivers the sealant.

An additional embodiment of the present invention utilizes an inflatable balloon as an insertion stop. Instead of being located outside of a body vessel, it is positioned inwardly of the vessel wall. After being so positioned through the puncture of the vessel wall, the balloon is inflated and the sealant is delivered at the puncture. The balloon blocks the vessel puncture and prevents the sealant from entering the vessel puncture.

In still another embodiment, the sealant is delivered without the use of an insertion stop. In this embodiment, the apparatus is inserted through an incision or puncture in the skin tissue. After being positioned in the proper location like a fatty area, such as a breast, the sealant is delivered through apertures formed adjacent to the distal end of the apparatus. This particular apparatus has utility for controlling body fluid discharge and for plugging or otherwise filling cavities that result from a medical procedure, such as involving a tissue biopsy.

Based on the foregoing summary, a number of advantages of the present invention are readily discerned, a medical apparatus is provided for delivery of a sealant to control body fluid and/or serve as a filler for a body cavity created as a result of a tissue biopsy. With regard to its use for sealing a puncture, the apparatus can be readily manipulated for delivering the sealant to the desired location. Patches that are difficult to manipulate are not needed. Necessary pressure for controlling the positioning and maintaining of the sealant is readily accomplishable by directly using fingers of the practitioner or, alternatively, using a pressure applicator device that is able to forcefully engage the skin tissue adjacent to the body area of interest. The sealant that is utilized can be a single component or is, preferably, a composition that includes thrombin and fibrinogen. The apparatus is able to provide a desired mixing of these two components.

Other features and benefits of the present invention will become apparent from the accompanying figures and the detailed description following hereinafter.
Fig. 1 is a perspective view of the apparatus of the present invention;
Fig. 2 is a longitudinal cross-sectional representation of the apparatus;
Fig. 3 is a transverse cross-sectional representation of the apparatus taken along lines 3-3 of Fig. 2;
Fig. 4 is a transverse cross-sectional representation of the apparatus taken along lines 4-4 of Fig. 2;
Fig. 5 illustrates the orientation of the apparatus of the present invention when inserted into a patient;
Fig. 6 is a cross-sectional representation of a body site of a patient having an introducer providing external access to an artery;
Fig. 7 illustrates the insertion of a guide wire through the introducer into the artery;
Fig. 8 illustrates the subsequent removal of the introducer from the patient;
Fig. 9 shows the apparatus threaded longitudinally onto the guide wire;
Fig. 10 illustrates the insertion of the apparatus into the arterial puncture via the use of the guide wire;
Fig. 11A shows the commencing of injecting sealant into the patient at a distance from the arterial puncture site;
Fig. 11B presents the same configuration as Fig. 11A with the exception that the guide wire has not been removed from the apparatus;
Fig. 12A shows the distribution of the sealant once an effective amount of the sealant has been injected into the patient to seal the puncture of the artery;
Fig. 12B is analogous to Fig. 12A with the exception that the guide wire remains within the apparatus;
Fig. 13A illustrates the removal of the guide wire prior to removing the apparatus from the patient;
Fig. 13B shows the removal of the guide wire and the apparatus simultaneously from the patient;
Fig. 14 illustrates the application of pressure to the patient body site once the apparatus and the guide wire have both been removed from the patient;
Fig. 15 illustrates the sealing of the arterial puncture;
Fig. 16A is a representation, similar to Fig. 11A, but using the pressure applicator device to apply a force to the patient's skin during delivery of the sealant;
Fig. 16B is a view, similar to Fig. 13A, illustrating removal of the apparatus while still applying pressure to the skin using the pressure applicator device;
Fig. 17 is a perspective view of another embodiment of an apparatus for delivery of a sealant to a body site having a puncture that utilizes an inflatable balloon adjacent to the distal end of the apparatus;
Fig. 18A illustrates the apparatus of Fig. 17 being inserted, using an opterator, adjacent to a body site having a puncture to be sealed;
Fig. 18B illustrates the apparatus of Fig. 17 with the opterator removed and the distal end of the apparatus being positioned before inflation of the catheter balloon or insertion stop;
Fig. 18C illustrates the inflation of the catheter balloon to prevent the sealant from passing through the puncture into the body vessel;
Fig. 18D illustrates removal of the apparatus for delivering of the sealant while the pressure applicator device remains stationary applying pressure to the skin tissue;
Fig. 19A illustrates yet another embodiment of the invention for delivery of sealant adjacent to a body cavity after, for example, a tissue biopsy; and
Fig. 19B illustrates the removal of the apparatus of Fig. 19A but maintaining a pressure applicator device against the skin for a short period of time.

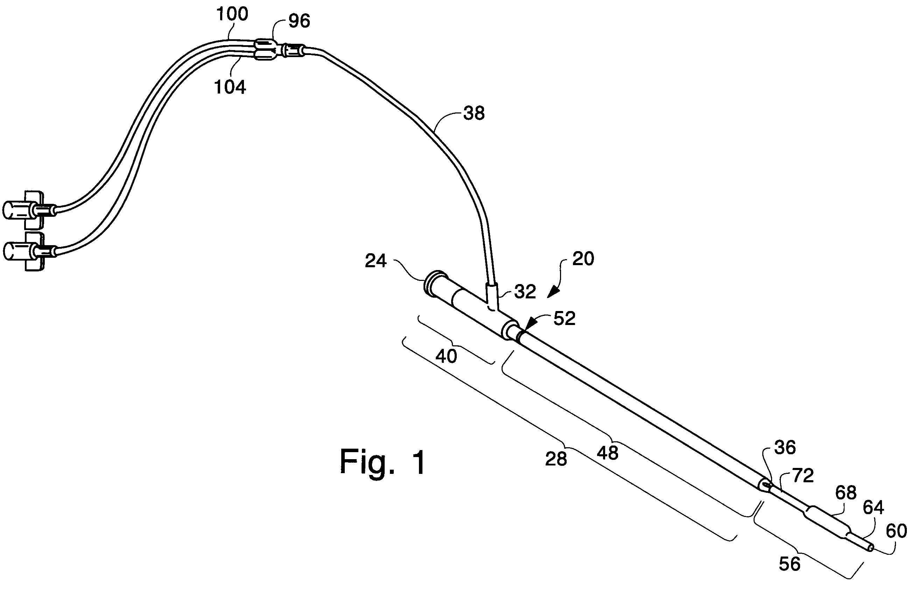

Fig. 1 provides a perspective view of the hemostatic apparatus 20 of the present invention. Proceeding from the apparatus end 24, which remains external to the patient's body, an apparatus trunk 28 is provided which constitutes the majority of the length of the apparatus 20. The trunk 28 provides a sealant input channel 32 for inputting a hemostatic sealant 34 via tubing 38. Preferably, the sealant 34 may be created from two components, which require mixing in a mixing chamber 96 prior to injection.

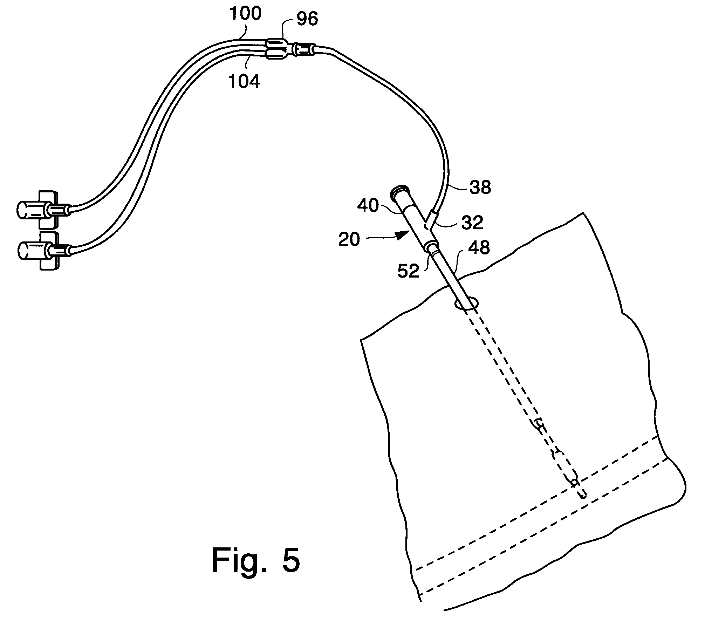

Additionally, trunk 28 provides at least one interior chamber (discussed below) for conveying the hemostatic sealant 34 from the sealant input channel 32 to one or more sealant dispensing apertures 36 which are located internal to the patient when the sealant 34 exits the apertures for sealing an arterial puncture. The trunk 28 can be defined as including (a) an upper section 40 having the sealant input channel 32 and (b) a lower section 48 having the sealant dispensing apertures. The upper section 40 is preferably formed as a single piece of rigid material, such as a rigid plastic, for ease of manipulation of apparatus 20 by a medical practitioner. The lower section 48 is fused or glued to the upper section 40 at seam 52, the lower section 48 being substantially cylindrical. The lower section 48 preferably consists of a flexible material such as polyvinyl chloride. Such flexibility is advantageous in the lower section 48 since, when the apparatus 20 is positioned within a patient (e.g., Fig. 5), the end of lower section 48 having apertures 36 is internal to the patient while the opposing seam 52, remains external to the patient. This flexibility facilitates use by the practitioner and affords greater comfort for the patient when the apparatus 20 is used.

Attached to the end of lower section 48 having apertures or outlets 36 is a stem 56 having three geometrically distinct segments along its length traversing from apertures 36 to stem end 60. Commencing from the stem end 60, the segments are, respectively, an insertion tip 64 for entering an arterial puncture; an insertion stop 68 having larger diameter than the puncture such that only the insertion tip 64 is able to enter the artery or lumen to be patched; and a spacing tube 72 which provides the remainder of the spacing, preferred for the present invention, between the insertion tip 64 and the apertures 36. Note that the stem 56 is also preferably a single molded piece of flexible material and that the stem 56 and the lower portion 48 may be fused together as a single integrated unit. Regarding the spacing or distance with reference to the apertures 36, in order to properly and accurately deliver the sealant 34, the distance between the apertures 36 and the distal end 74 of the insertion stop 68 should be between about 0.125 inch and 1 inch.

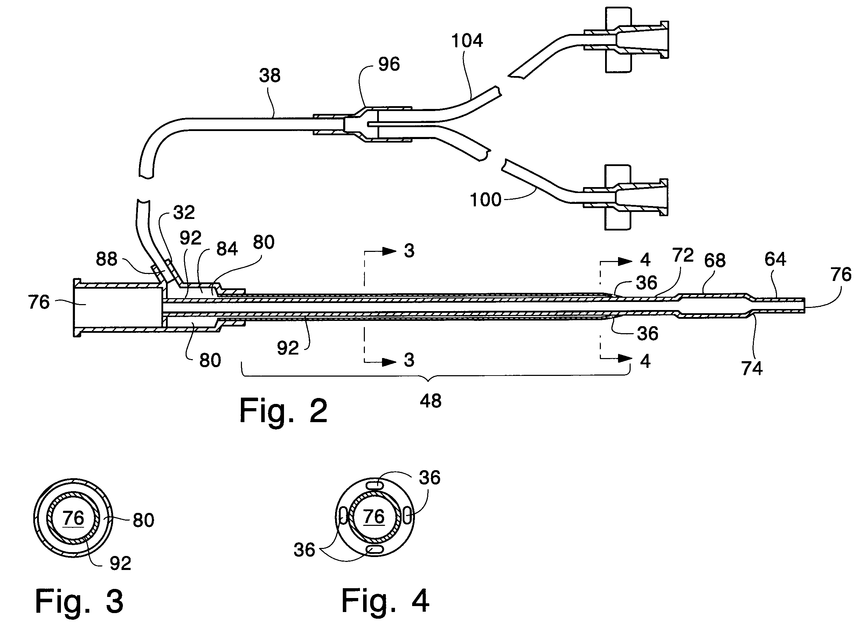

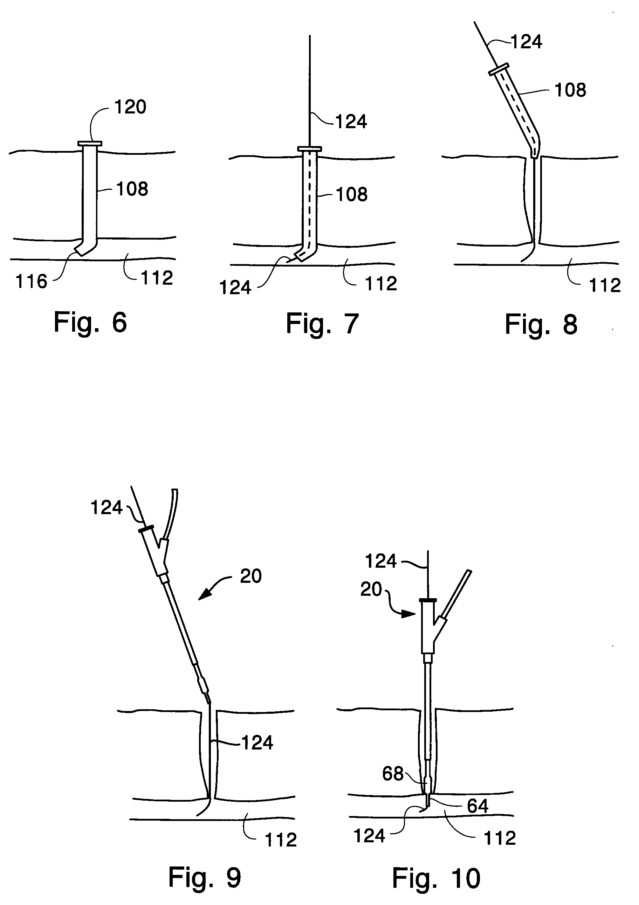

Referring now to Fig. 2, the internal structure of the apparatus 20 is presented. Centrally located along the length of the device 20 is a guide bore 76. The guide bore 76 is used in conjunction with an arterial guide wire or thread 124 (as shown in, for example, Fig. 9) so that when the arterial guide wire is threaded through the guide bore 76 from the stem end 60, the apparatus 20, and more particularly the insertion tip 64, can be reliably guided into the arterial puncture to be sealed. In addition to the guide bore 76, the trunk 28 includes one or more sealant delivery chambers 80 which deliver the hemostatic sealant 34 from the sealant input channel 32 to the apertures 36 which are best illustrated in Fig. 4. Thus, each such sealant delivery chamber 80 has an upper end area 84 open to the internal bore 88 of the sealant input channel 32. In addition, each sealant delivery chamber 80 is also open to an aperture 36 for injecting the sealant 34 into the patient. Moreover, note that partition 92 entirely separates the sealant delivery chambers 80 from the guide bore 76 (Fig. 3). In the present embodiment, the partition 92 is tubular in shape and its lower end, adjacent apertures 36, may be integrally formed with stem 56 such that spacing tube 72 can be considered an extension of the partition 92.

As discussed in reference to Fig. 1, the sealant 34 may be composed of two or more components which require mixing immediately prior to use. In one preferred operation of the apparatus 20 where two sealant components are used, the mixing chamber 96 is used to mix the two sealant components contained in tubes 100 and 104, respectively. Further, it is preferred that one of the two components (contained in one of the tubes 100 or 104) include thrombin and the other component (contained in the other tube 100 or 104) include fibrinogen. Alternatively, it is within the scope of the present invention to also use a pre-prepared sealant 34. In such an alternative embodiment, the pre-prepared sealant may be injected into the tubing 38 from a single conventional syringe, thus precluding the need for a mixing chamber 96.

The operation of the apparatus 20 is illustrated in Figs. 6-14. Fig. 6 shows an arterial catheter or introducer 108 which is tubular in nature having an open end 116 internal to the artery 112 and an opposite open end 120 external to the patient, thereby maintaining access to the artery blood flow from outside the patient. Prior to removal of introducer 108 from the patient, an arterial guide wire is introduced into the catheter 108, as shown in Fig. 7. Following this, in Fig. 8, the introducer 108 is removed from the patient and, as shown in Fig. 9, the arterial guide wire 124 is threaded through the guide bore 76 of device 20. Subsequently, in Fig. 10, apparatus 20 is guided through the catheter opening where the introducer 108 previously resided until the insertion tip 64 enters the artery 112 through the arterial puncture and there is resistance to further entry due to the insertion stop 68 preventing the device 20 from proceeding further into the artery. Following this step, the arterial guide wire 124 may be removed from the patient.

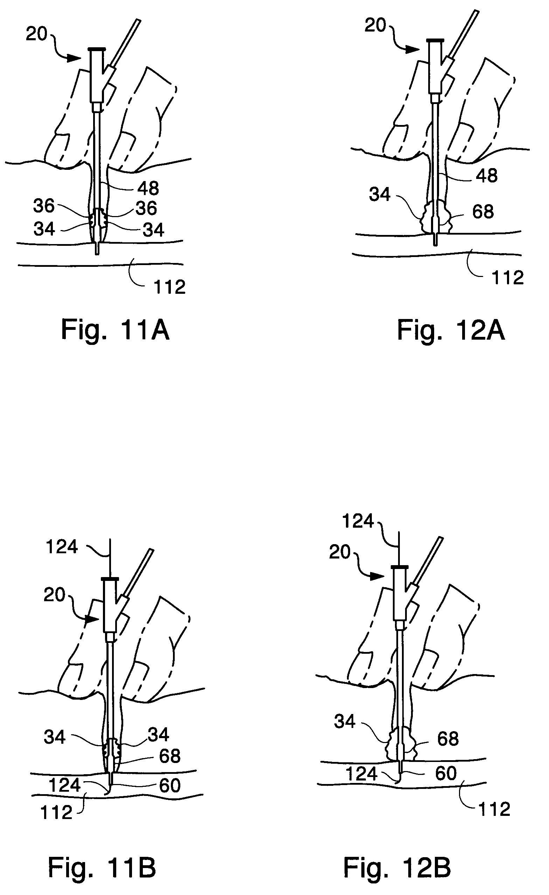

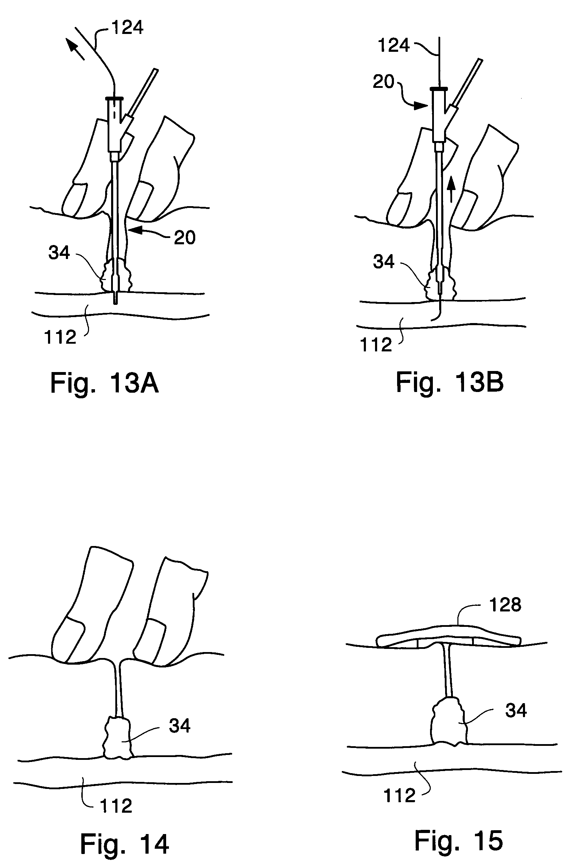

Subsequently, as shown in Fig. 11A, the sealant 34 is urged from the apertures 36 in the general direction of the arterial puncture. Further note that manual pressure about the catheter opening may be initiated at this time as depicted by the dash fingertips in this figure. Referring now to Fig. 12A, as the sealant 34 exits the apparatus 20 it forms a substantially single viscous mass which is tissue adhesive. Also as depicted in this figure, pressure is applied on the external body site adjacent the catheter opening in order to facilitate the sealant's flow such that the sealant substantially completely surrounds the insertion stop 68. Following this step, as shown in Fig. 14, the apparatus 20 is removed from the patient. Further note that it is preferred that continuous external pressure be applied once the apparatus 20 is removed from the catheter opening for a period of approximately two minutes or less, thereby assuring an effective patch of the arterial puncture by the sealant 34. Thus, as shown in Fig. 15, the external pressure is removed and the catheter site is provided with a bandage 128 to prevent infection.

As an alternative to the above method, Figs. 11B and 12B depict steps analogous to the steps depicted in Figs. 11A and 12A, respectively, however, the guide wire 124 remains in place within the apparatus 20 while the sealant 34 is being injected into the patient. In this alternative method of operation, the guide wire 124 may subsequently be removed either before the apparatus 20 is removed, as in Fig. 13A, or alternatively the guide wire 124 can be removed concurrently with the apparatus 20, as depicted in Fig. 13B. However, note that regardless of which of these latter methods of operation are performed, subsequent to the steps depicted in either of Figs. 13A or 13B, the steps corresponding to Figs. 14 and 15 are performed.

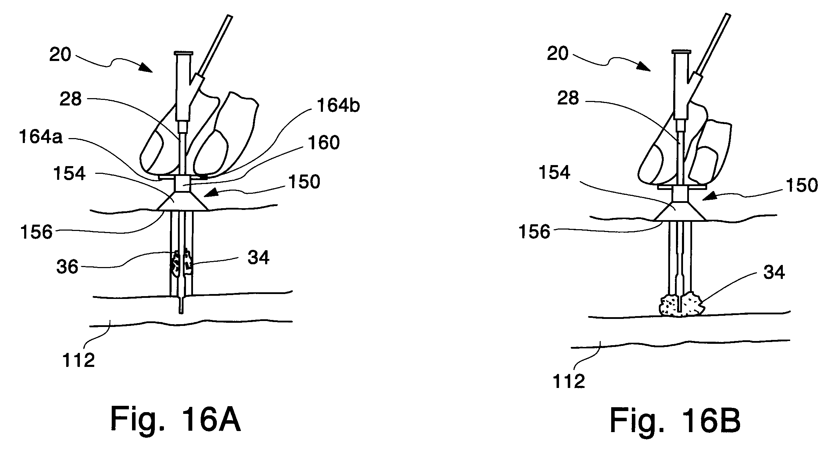

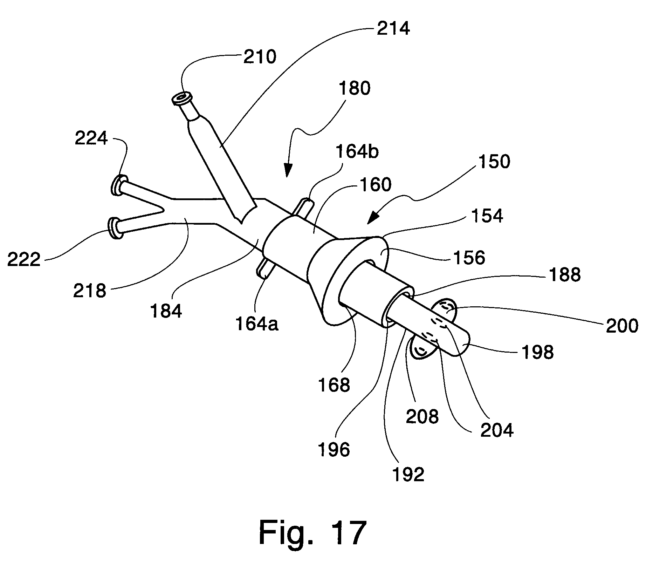

With reference now to Figs. 16A and 16B, a variation of the embodiment of Fig. 11A is illustrated. Instead of the medical practitioner's fingers directly engaging the skin tissue adjacent the body site of interest, a pressure applicator device 150 forcefully engages the skin during the delivery of the sealant 34 adjacent to the body puncture. Referring also to Fig. 17, the pressure applicator device 150 includes a pressure applying member 154, that is frusto-conical in shape, and with an annular surface 156 for contacting the skin. Integral with the pressure applying member 154 is a body member 160 having a free end adjacent to which gripping wings 164a, 164b are attached or formed. Extending through the pressure applying member 154 and the body member 160 is a bore 168 (Fig. 17), which is of a diameter or size so that it can receive, and be positioned around, at least portions of the trunk 28 including the lower section 48.

In using the apparatus 20, in combination with the pressure applicator device 150, as seen in Fig. 16A, as the sealant 34 exits the apertures 36, the pressure applying member 156 forcefully engages the skin using the force applied by the fingers of the medical practitioner to the wings 164a, 164b. The force applied is sufficient to influence the positioning of the sealant 34 at the desired location adjacent to the body puncture. As can be appreciated, it is desirable that as much of the surface area of the annular member 156 contact the skin as can be achieved to provide the desired pressure.

The pressure applicator device 150 can be moved relative to the apparatus 20. That is, as illustrated in Fig. 16B, after the sealant 34 has been properly delivered to the puncture, the apparatus 20 is removed while the pressure applicator device 150 remains in place to continue to apply pressure for the relatively short, desired time period.

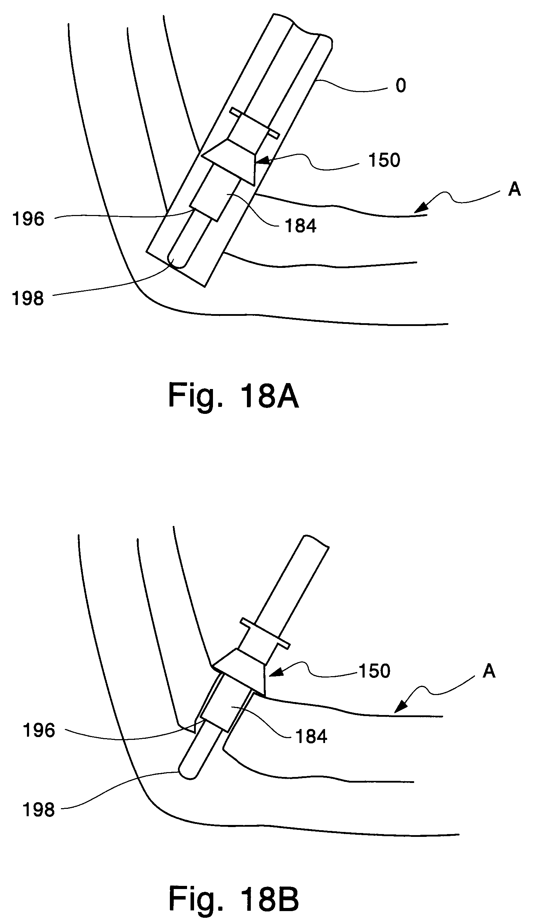

With continued reference to Fig. 17, as well as Figs. 18A-18D, a further embodiment of the invention for supplying a sealant to a body site having a puncture is described that may, but need not, include the pressure applicator device 150. As seen in Fig. 17, the apparatus 180 of this further embodiment has a tubular sleeve 184 that is hollow to define a sealant conduit 188. Held within the sealant conduit 188 is balloon catheter 192 having a size or diameter less than that of the sealant conduit 188 to permit sealant to exit the outlet or aperture 196 at the free end of the tubular sleeve 184. The balloon catheter 192 has an insertion end 198 at a free end thereof that is insertable through a puncture at a body site. An inflatable balloon 200 (shown inflated in Fig. 17) is provided at a proximal end of the insertion end 198. The balloon 200 is inflated using holes 204 formed in the balloon catheter 192. Since the balloon holes 204 communicate with the balloon 200. When deflated, the insertion end 198, together with the deflated balloon 200, are received through the wall of a body vessel, such as an artery. The balloon 200 is spaced from the outlet 196 by a desired distance, like in the apparatus 20. The desired distance between the outlet 196 and a proximal end 208 of the balloon 200 is comparable to the similar distance defined with respect to apparatus 20, i.e., in the range of .125 of an inch to 1 inch.

In connection with inflating the balloon 200, a port 210 is provided for receiving a liquid substance, such as a sterile saline mixture. The port 210 communicates with a liquid passageway member 214, which is joined to the tubular sleeve 184. The outlet of the liquid passageway member 214 communicates with the balloon catheter 192. When desirable and appropriate to enlarge the balloon 200, the liquid is supplied to the port 210 and is removed when it is desirable to remove the liquid from the balloon 200. Like the previous embodiment, the apparatus 180 has a sealant delivery member 218 that is operatively joined to the tubular sleeve 184. In a preferred embodiment, the sealant 34 is comprised of the two components, thrombin and fibrinogen, which are introduced separately using a first sealant port 222 and a second sealant port 224. The two component sealant is mixed using the sealant delivery member 218. When the sealant 34 is delivered for purposes of sealing or patching, it is caused to move or flow through the tubular conduit 188 and subsequently exits from the outlet 196.

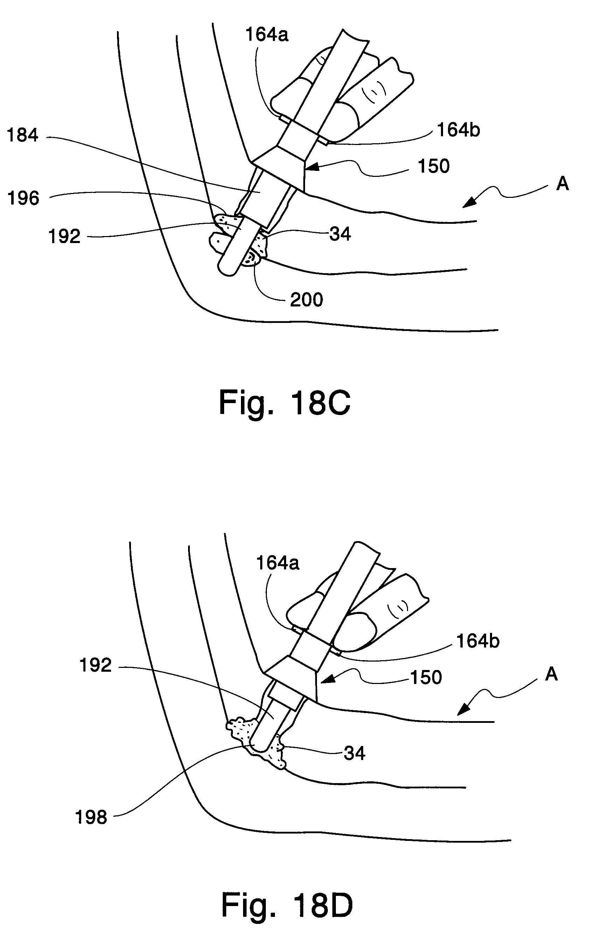

With respect to the operation of the embodiment of Fig. 17, it has particular utility in sealing a puncture in a body site at a vascular graft, which is attached to a vein and an artery. Such a puncture may be the result of a medical dialysis procedure conducted using a patient's arm. After such a procedure, it is necessary to stop unwanted bleeding. In using the apparatus 180, as part of the dialysis procedure, an opterator O is in place and positioned through the skin tissue of the patient's arm A. The apparatus 180 is of a size to be inserted into and along the opterator O. The opterator O acts as a guide for the apparatus 180 so that it can be accurately positioned for use in delivering a sealant to control blood flow from the puncture. After the apparatus 180 is properly positioned, with the insertion end 198 and the balloon 200 of the balloon catheter 192 located past or beyond the body vessel or graft wall, the opterator O is removed from the patient's arm A, as seen in Fig. 18B. Next, as illustrated in Fig. 18C, the balloon 200 is supplied with the liquid and expands. The enlarged balloon 200 is located inwardly of the body vessel wall and prevents sealant from entering the body lumen. After the balloon 200 is enlarged, the sealant 34 is delivered and moves along the conduit 188 and exits the outlet 196. As illustrated in Fig. 18C, none of the sealant is able to pass into the body lumen. At the same time the sealant 34 is escaping from the outlet 196, pressure is being applied to the outer skin adjacent to the puncture that is being patched. In this embodiment, such pressure is applied using the pressure applicator device 150. This assists in assuring that the sealant 34 is properly positioned for providing the desired patch in order to, for example, stop unwanted body vessel bleeding in the arm A.

After the sealant has been delivered for controlling the bleeding, as illustrated in Fig. 18D, the apparatus 180 is removed from the arm A. In that regard, the liquid in the balloon 200 of the balloon catheter 192 is removed so that the insertion end 196 can be removed past the vessel wall. The viscous sealant collapses or covers the space that was previously occupied by the balloon catheter 192, but does not pass into the body lumen. During the time the apparatus 180 is being removed, the pressure applicator device 150 is utilized to maintain pressure to influence the positioning of the sealant 34. As can be appreciated, instead of using the pressure applicator device 150, the medical practitioner could have used his/her fingers pressed against the skin adjacent to the body site of interest.

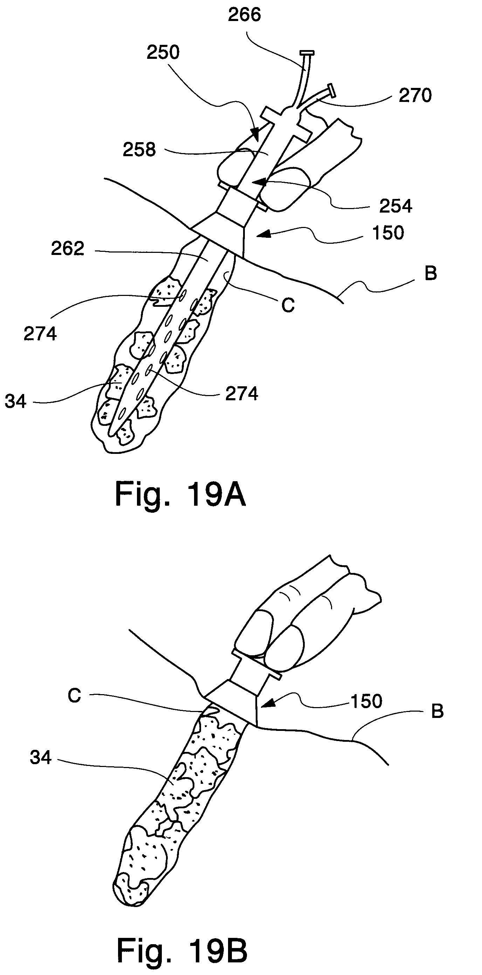

In some embodiments for delivery of a sealant to a body site, it may not be necessary or appropriate to utilize a stop or blocking member, such as the insertion stop 68 or the balloon 200. With reference to Figs. 19A-19B, an apparatus is disclosed for supplying the sealant 34 to a body cavity. In one application, the body cavity C results from a medical procedure, such as a biopsy, that was performed on a patient. Such a biopsy may be performed, for example, in a patient's breast B. At the completion of the biopsy, body fluid tends to discharge and must be controlled, as well as filling the body cavity C. This function is accomplished using the apparatus 250, which includes a tubular sealant carrier 254 that can be defined as having a proximal section 258 and a distal section 262. The proximal section 258 communicates with first and second sealant ports 266, 270 that receive sealant components that are mixed together in the proximal section 258. As should be appreciated, like the other embodiments, single component sealants 34 can also be utilized. The distal section 262 has a length for extending into the body cavity C and is typically formed integral with the proximal section 258. The distal section 262 may be made of any length and the length thereof depends upon its particular application and/or the body make-up of the patient. For example, in connection with a particular medical procedure, a patient having greater fat tissue would probably require a longer distal section 262. The distal section 262 has a number of outlets or apertures 274 through which the sealant 34 passes for controlling body fluid discharge and/or filling the body cavity C.

In using the apparatus 250, after the medical procedure, such as the biopsy, the distal section 262 is inserted into the body cavity C that resulted from the biopsy procedure. After proper manipulation to position the distal section 262 with outlets 274 in the proper place in the body cavity C, the sealant components are delivered through the first and second sealant ports 266, 270 into the sealant carrier 258. The sealant 34 moves from the proximal section 258 to the distal section 262 and exits the outlets 274 into the body cavity C. As with the other embodiments, during the delivery of the sealant 34 to the body cavity C, exterior pressure is applied to the skin, such as at the breast B outer wall. In the embodiment illustrated, the pressure applicator device 150 is being used by the practitioner to provide the necessary force, although it should be appreciated that other means could be utilized, such as the fingers of the practitioner.

With reference to Fig. 19B, after a desired amount of sealant 34 has been delivered to the body cavity C, the apparatus 250 is removed, while the pressure applicator device 150 remains in use. As the apparatus 250 is removed, the viscous sealant 34 occupies the space previously occupied by the proximal section 262. The medical practitioner continues to provide a force at the body cavity for the relatively short period of time while the sealant is curing or hardening.

Even though particular medical procedures have been described, it should be understood that the apparatuses of the different embodiments may be utilized in other different medical procedures for similar sealant purposes. It should also be appreciated that certain variations of these embodiments can be readily achieved including, in the case of the embodiment of Fig. 17, permitting relative movement between the tubular sleeve 184 and the balloon catheter 192. The defined parts of each apparatus can be made of appropriate materials and have varying sizes including, for example, the balloon 200 of the balloon catheter 192 being made of any suitable or proper inflatable material, such as a latex or a polyurethane material.

## Claims

1. An apparatus for sealing a puncture in a body, comprising:
insertion means for entering the puncture;
stopping means connected to said insertion means, said stopping means being adjacent to the puncture when said apparatus is used in sealing the puncture;
trunk means connected to said stopping means for manual manipulation by a user of the apparatus;
sealant delivery means communicating with said trunk means and including at least a first sealant dispensing outlet; and
a sealant that is urged to exit said first outlet and in which said stopping means prevents said sealant from entering the puncture but said stopping means is removed from adjacent the puncture after said sealant is located adjacent the puncture.

2. An apparatus, as claimed in Claim 1, wherein:
said stopping means is positioned outwardly of the puncture when said apparatus is used in closing the puncture.

3. An apparatus, as claimed in Claim 1, wherein:
said stopping means includes an expandable balloon and is positioned inwardly of the puncture.

4. An apparatus, as claimed in Claim 1, wherein:
said sealant forms a majority of the seal.

5. An apparatus, as claimed in Claim 1, wherein:
said sealant is bioabsorbent and forms substantially a single viscous mass with a tissue adhesive surface upon exiting said first sealant dispensing outlet.

6. An apparatus, as claimed in Claim 1, wherein:
said stopping means includes an end and said first outlet is spaced from said end of said stopping means by a desired distance in the range of about .125 - 1 inch.

7. An apparatus, as claimed in Claim 1, wherein:
a guide bore is provided through said insertion means and said trunk means so that by threading an elongate puncture locating device through said guide bore, said insertion means is guided in entering the artery through the puncture.

8. An apparatus, as claimed in Claim 1, further including:
a pressure applicator device located outwardly of said trunk means for use in applying pressure adjacent to the puncture.

9. An apparatus for providing a sealant to a body site, comprising:
a sealant carrier having a proximal section and a distal section and having a length sufficient to extend into the body for a desired distance, said distal section having at least a first outlet;
a sealant port connected to said sealant carrier; and
a sealant that is received in said sealant carrier through at least said sealant port, said sealant being viscous and movable into said distal section and in which at least some of said sealant exits said first outlet, wherein said sealant occupies space that was occupied by at least portions of said distal section after said distal section is removed from the body.

10. A method for closing a puncture in a body lumen, comprising:
providing sealant in a puncture sealing apparatus, said apparatus having at least a first sealant dispensing outlet;
entering the puncture, via an adjacent body site, with an insertion segment of said puncture sealing apparatus;
using an insertion stop to prevent further entry of said puncture sealing apparatus into the lumen;
injecting said sealant through said first outlet for forming a patch for the puncture while using said insertion stop to prevent said sealant from entering the puncture;
removing said puncture sealing apparatus including said insertion stop from the body site.
